# EUROPEAN PATENT SPECIFICATION

(11) **EP 3 054 777 B1**
(45) Date of publication and mention of the grant of the patent: **07.03.2018**
(21) Application number: 14781196.2
(22) Date of filing: 07.10.2014
(51) Int. Cl.: A23D 9/00, A23D 9/007, A23D 9/013, A23K 20/158, A23L 33/115

(54) **EDIBLE LIPID COMPOSITION COMPRISING STEARIDONIC ACID AND OLIVE OIL**
ESSBARE LIPIDZUSAMMENSETZUNG MIT STEARIDONSÄURE UND OLIVENÖL
COMPOSITION LIPIDIQUE COMESTIBLE COMPRENANT DE L'ACIDE STÉARIDONIQUE ET DE L'HUILE D'OLIVE

(30) Priority: 07.10.2013 EP 13187560
(43) Date of publication of application: 17.08.2016
(73) Proprietor: Zinzino AB, 421 31 Västra Frölunda (SE)
(72) Inventor: HOFSTRA, Harmen, 3972 PP Driebergen (NL); SAELE, Ørjan, 1367 Snarøya (NO); EIDE, Ola, 1356 Bekkestua (NO); SAGA, Linda Christine, 3914 Porsgrunn (NO)
(74) Representative: Plougmann Vingtoft a/s
(86) International application number: PCT/EP2014/071418
(87) International publication number: WO 2015/052171

(56) References cited:
- EP-A2- 0 696 453
- WO-A1-2008/085841
- AU-A8- 2012 100 994
- US-A1- 2007 207 223
- US-A1- 2009 099 261
- US-A1- 2012 231 142

## Description

### Technical field of the invention

The present invention relates to an edible lipid composition comprising olive oil and a stearidonic acid component (SDA component), wherein said SDA component comprises stearidonic acid in an amount of at least 6% by weight and wherein said olive oil comprises polyphenols in an amount of at least 250 mg per kg olive oil and wherein the ratio between the olive oil and the SDA component is from 3:8 to 3:2. Further, the present invention relates to a method of preparing such lipid composition and the use of such lipid composition for use in preventing or reducing the risk of developing cardiovascular diseases, coronary thrombosis, and other inflammatory diseases such as atheroschlerosis, cancer, diabetes, psoriasis, arthritis, rheumatism and astma, osteoroporosis, and alzheimers.

### Background of the invention

Life-style related health problems are becoming an increased threat to the population. Atherosclerosis, cardiovascular diseases, cancer, and diabetes are examples of such life-style related conditions.

Atherosclerosis is the build-up of a waxy plaque on the inside of blood vessels and may also be referred to as hardening of the arteries. Atherosclerosis, which may also be termed arteriosclerosis, is a progressive process and is responsible for most heart disease. The plaque deposits on the blood vessels will block the flow of the blood and may therefore lead to blood thrombosis or other related diseases or conditions.

Atherosclerotic injuries are formed when three circulation components, monocytes, blood plates, and T-lymphocytes reacts with low-density lipoproteins (LDL-cholesterol) and two cell types in the artery wall, endothelialcells (EC) and the smooth muscle cells (SMC). Thus, a high level of LDL-cholesterol in the blood increases the risk of developing atherosclerosis; while high levels of high-density lipoprotein (HDL-cholesterol) decreases the risk of develop coronary artery diseases, i.e. atherosclerosis.

The precursor of atherogenesis is recruitment of monocytes and lymphocytes from the peripheral blood to intima in the vessel walls, a condition which is believed to be dependent of high levels of LDL. When LDL accumulates, bound lipid and protein becomes oxidized and glycosylated. Cells in the vessel wall seem to understand this change as a dangerous signal and ask for enhancement from the body's defense system. These processes seem to enhance an upregulation of adhesion molecules on endocells, particularly the vascular cell adhesion molecule-1 (VCAM-1) and the intracellular adhesion molecule-1 (ICAM-1). In this way the recruitment of monocytes and lymphocytes is initiated, leading to increased transmigration of monocytes, up-regulated exposure of adhesion molecules on the endothel, and production and release of chemical attractant substances. Available modified LDL is necessary for the further development of macrophages into foam cells, which is the main reason in development of fat deposits under the vessel walls endothel.

As mentioned above, it is known that monocytes play a central role in the early phase of atherogenesis. One of the first events in the atherosclerotic process is mobilisation of monocytes into intima. Exactly how the functional characteristics of circulating monocytes are related to atherogenesis is complex and not fully understood in details, but that hyperactive monocytes are crucial in relation to rheumatism, psoriasis, and other inflammatory diseases is generally known.

Thus, the levels of LDL and HDL in the blood are known to play a role in developing cardiovascular diseases and other inflammatory diseases. Further, it is known that ingestion of the long chained polyunsaturated omega-3 fatty acids, eicosapentaenoic acid (EPA), and docoxhexaenoic acid (DHA) can increase the HDL levels in blood and therefore has an influence in preventing development of these types of diseases.

During the last decades, intake of Omega-6 polyunsaturated fatty acids has increased in the Western diet, causing an imbalance in the Omega-6/ Omega-3 ratio. The arachidonic acid/eicosapentaenoic acid (AA/EPA) ratio of cell membranes is a mirror of the Omega-6/Omega-3 ratio in our diet. This ratio has constantly increased in Europe from 1:1 in 1850 to 15:1 or more in 2000, which is believed to affect our health in a negative way, such as increased susceptibility to lifestyle related health problems. Target for a healthy Omega-6/Omega-3 fatty acid balance should be 1:1-3:1. The inventors internal results of more than 17 thousand people in the Nordic contries show that the AA/EPA ratio of circulating red blood cells is around 12:1 in average, and the average omega-3 index (EPA+DHA) is 4%. The recommended ratio is 1:1-3:1 and omega-3 index above 8% for optimal health.

Yet our body cannot generate Omega-3s itself. Instead the Omega-3s must be obtained through your diet. The body must convert the short-chain version, alpha-linolenic acid (ALA), which is present in high amount in plant oils, to a long-chain version (EPA, DHA) to make use of it, but this conversion of ALA to EPA and DHA does not happen efficiently. Thus, the body has a need of supply of the long chained omega-3 fatty acids, EPA and DHA from other sources than ALA in plant oils.
It is an overall impression that dietary intakes of long chain omega-3 fatty acids are well below current recommended levels for optimal cardiovascular health. Fish and fish oil contain high levels of EPA and DHA and whilst an adequate intake of the long chain omega-3 fatty acids eicosapentaenoic acid (EPA) and docosahexaenoic acid (DHA) can be achieved by eating fatty fish at least 1-2 times per week, (equivalent to 250-500 mg per day of EPA and DHA), the majority of the population fails to achieve such intake.
A recommended amount of EPA and DHA may be obtained from fish or fish oil, which can prevent development of cardiovascular disease and other lifestyle related conditions. However, for vegetarians and vegans fish or fish oil are not an option and obtaining an effective amount of EPA and DHA can therefore be a challenge. In India, for example, about 40% of the population are vegetarians and therefore there is a need for a supplement of EPA and DHA not arriving from animals, including fish and fish oil. Further, fish oil has a characteristic smell and taste which is disliked by many. Thus, for this reason, an alternative to fish oil for obtaining EPA and DHA is also wished.

AU2012100994 discloses an edible lipid composition comprising olive oil and hemp seed oil. Hemp seed oil contains in average up to 2% by weight of stearidonic acid, which can be converted to EPA and DHA.

Hence, there is a need in the art for a composition which can supply the essential long chained polyunsaturated fatty acids, EPA and DHA, from a vegetable source in order for vegetarians and vegans to obtain the recommended amount of EPA and DHA the body needs, and a composition that will safeguard the oxidative protection of EPA and DHA in the body in order to prevent oxidative stress.

### Summary of the invention

Thus, an object of the present invention relates to providing a lipid composition which can ensure that the body obtains the daily amounts of EPA and DHA in a protected manner, recommended by authorities in order to have an effect in preventing artherosclerosis, cardiovascular diseases, and other inflammatory diseases, and wherein the composition is derived from plant sources and industrial sources of the Omega-3 component. Further, it is an object of the present invention to provide a lipid composition with an acceptable oxidative stability, i.e. to protect both blood lipids and lipids in the lipid composition from oxidation.

In particular, it is an object of the present invention to provide a lipid solution that solves the above mentioned problems of the prior art.

Thus, one aspect of the invention relates to an edible lipid composition comprising olive oil and a stearidonic acid component (SDA component), wherein said SDA component comprises stearidonic acid in an amount of at least 6% by weight, and wherein said olive oil comprises polyphenols in an amount of at least 250 mg per kg olive oil and wherein the ratio between the olive oil and the SDA component is from 3:8 to 3:2.

Another aspect of the present invention relates to a method of preparing the lipid composition according to the present invention, wherein the method comprises mixing of an olive oil comprising polyphenols in an amount of at least 250 mg/kg and a SDA component comprising at least 6% SDA and optionally a further oil, preferably the olive oil is selected to protect long chain fatty acids from oxidizing.

Yet another aspect of the present invention is to provide an edible lipid composition according to the present invention for use in administration to an animal or human for preventing or reducing the risk of developing cardiovascular diseases, coronary thrombosis, atherosclerosis, cancer, diabetes II, alzheimers, arthritis, rheumatism, osteoporosis, psoriasis, or astma.

### Brief description of the figures

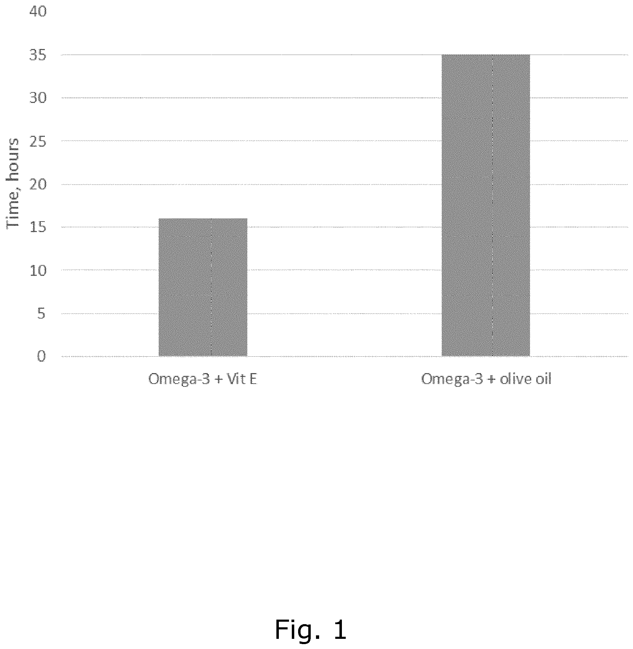

Figure 1 shows the antioxidative effect of polyphenols on omega-3 fatty acids, EPA and DHA.

### Detailed description of the invention

An aspect of the invention relates to an edible lipid composition comprising olive oil and a stearidonic acid component (SDA component), wherein said SDA component comprises stearidonic acid in an amount of at least 6% by weight, and wherein said olive oil comprises polyphenols in an amount of at least 250 mg per kg olive oil and wherein the ratio between the olive oil and the SDA component is from 3:8 to 3:2.

### Definitions:

Prior to discussing the present invention in further details, the following terms and conventions will first be defined:

In the context of the present invention, mentioned percentages are by weight (weight/weight) percentages unless otherwise stated.

The term "and/or" used in the context of the "X and/or Y" should be interpreted as "X", or "Y", or "X and Y".

Numerical ranges as used herein are intended to include every number and subset of numbers contained within that range, whether specifically disclosed or not. Further, these numerical ranges should be construed as providing support for a claim directed to any number or subset of numbers in that range. For example, a disclosure of from 1 to 10 should be construed as supporting a range of from 1 to 8, from 3 to 7, from 4 to 9, from 3.6 to 4.6, from 3.5 to 9.9, and so forth. All references to singular characteristics or limitations of the present invention shall include the corresponding plural characteristic or limitation, and vice versa, unless otherwise specified or clearly implied to the contrary by the context in which the reference is made.

In the context of the present invention, the term "ratio" by weight (weight/weight) refers to the ratio between the weights of the mentioned compounds. For example, a composition comprising 60 g olive oil and 40 g SDA component would have a weight ratio which is equal to 60:40, which is equal to 3:2 or 1.5 (that is 3 divided with 2), corresponding to 60% olive oil and 40% SDA component. Similarly, a mixture of 50 g olive oil and 50 g SDA component would have a ratio by weight of olive oil and SDA component of 50:50, which is equal to 1:1 or 1 (that is 1 divided with 1).

Unless defined otherwise, all technical and scientific terms used herein have the same meaning as commonly understood by one skilled in the art.

### Lipid composition:

The lipid composition according to the present invention is edible. By edible is meant that the lipid composition is suitable for being consumed. Thus, the edible lipid composition is a composition suitable for being eaten by an animal or human being without being sick and/or violating internal organs.

In the context of the present invention, the term "lipid" refers to one or more lipids and may be in the form of triglycerides, an oil, fatty acids, a concentrate or a fat.

By the term "lipid composition" is meant a composition comprising a substantial part of lipids, such as oils or fats.

In the context of the present invention, the term "substantially" refers to at least 90% lipids by weight, such as at least 90% oil by weight in the lipid composition. The lipid composition may for example comprise an amount of at least 92% lipid by weight, such as at least 93% lipid by weight, for example at least 95% lipid by weight, preferably at least 96% lipid by weight, such as at least 97% lipid by weight, even more preferably at least 98% lipid by weight, such as 99% lipid by weight.

In an embodiment of the invention, the edible lipid composition is liquid.

In an embodiment of the invention, the lipid composition is an oil.

Preferably, the lipid composition is a mixture of two or more edible oils.

In another embodiment of the invention, the lipid composition is a concentrate or a mixture of an oil and a concentrate, such as a mixture of olive oil and a concentrate comprising more than 6% SDA. The lipid composition may comprise one or more additional oils or fats or concentrates.

The lipid composition according to the present invention comprises olive oil and an stearidonic acid component (SDA component), but may comprise other sources of lipids, fats, oils, or free fatty acids in the form of ethylesters. In an embodiment of the invention, the lipid composition comprises one or more further oils.

The further lipid source other than olive oil and SDA component may be any lipid or fat source which is suitable for use in nutritional compositions to be fed to an animal or human, for example free fatty acids or some vegetable fats or oils. Also monoglycerides, diglycerides, and/or triglycerides may be added as a further oil.

In an embodiment of the invention, a further oil, such as for example an alga oil, may be added to the lipid composition according to the invention.

### SDA component:

The lipid composition according to the present invention comprises a stearidonic acid component, hereinafter referred to as a SDA component. The SDA component according to the present invention comprises stearidonic acid (SDA) in an amount of at least 6% by weight.

In an embodiment of the invention, the SDA component comprises SDA in an amount of at least 8% by weight, such as at least 10% by weight, preferably 10-15% by weight, such as 12-15% by weight of the SDA component.

In another embodiment, the SDA component comprises from 10 to 100% by weight SDA, such as from 10 to 80% by weight SDA, preferably from 12 to 70% by weight SDA, such as from 15 to 60% by weight SDA, preferably 15-40% by weight SDA.

Stearidonic acid (SDA) is an omega 3 fatty acid (C18:4) which can be converted to the beneficial polyunsaturated fatty acids eicosapentaenoic acid (EPA) and docosahexaenoic acid (DHA). Alpha-linolenic acid (ALA) which is present in plant oils in a high amount may also be converted to EPA and DHA, but the body is very poor in conversion of ALA to EPA and DHA. In general, ALA provides less than 4% of EPA and DHA in the fatty acid profile in the blood. On the contrary, it has been found out that SDA is more efficient converted to EPA and DHA. Without being bound by any theory, the inventors of the present invention believe that about 20-60% of SDA in an oil may be converted to EPA and DHA. This conversion rate is increased by addition of olive oil due to the protective effect of the olive oil on oxidative unstable omega-3 fatty acids. A better protection of omega-3 fatty acids will make more omega-3 available in the blood for in vivo health processes. When more SDA is available in the blood due to olive oils protective effect, the conversion rate of SDA to EPA and DHA will increase. Furthermore, sources with a high SDA content allow the body to convert SDA to EPA and DHA more efficiently, because it bypasses the rate limiting step of the ALA conversion process. ALA is the starting point of the convertion metabolism to EPA/DHA, where SDA is an imetermediate in the same metabolism. When bypassing the first rate limiting step (ALA step), the conversion of the intermediate step (SDA step) to EPA and DHA is increased.

As explained above, EPA and DHA have anti-inflammatory effects and also beneficial effects on preventing cardiovascular diseases, especially in combination with olive oil rich in stabilizing antioxidants, such as polyphenols. EPA and DHA have been known to be present in high amounts in fish and fish oils.
From Harris, W. et al "The Omega-3 idex: a new risk factor for death from coronary heart disease Prevention Meficine", 39, 2004, it is known that low intake or low blood levels of EPA and DHA are independently associated with increased risk of death from coronary heart disease (CHD). From Harris et al, it is also disclosed that fish or fish oil have been demonstrated to reduce CHD mortality at intakes of about 1 g/day.

Further, the European Food Safety Authority (EFSA) has concluded that it is safe to consume up to 5 g per day of marine omega-3 fatty acids (EPA and DHA). EFSA is responsible for approving health claims based on scientific evidence for bioactive compounds. The following health claims for EPA and DHA have been approved by EFSA.
- DHA and EPA contribute to the normal function of the heart (0.25 g per day)
- DHA and EPA contribute to the maintainance of normal blood pressure (3 g per day)
- DHA and EPA contribute to the maintenance of normal blood triglycerides (2 g per day)
- DHA contributes to maintenance of normal blood triglyceride levels (2 g per day in combination with EPA)
- DHA contributes to maintenance of normal brain function (0.25 g per day)
- DHA contributes to the maintenance of normal vision (0.25 g per day)
- DHA maternal intake contributes to the normal brain development of the foetus and breastfed infants (0.2 g DHA plus the daily recommended intake of omega-3 fatty acids (EPA and DHA) for adults which is 0.25 g per day).
- DHA maternal intake contributes to the normal development of the eye of the foetus and breastfed infants (0.2 g DHA plus the daily recommended intake of omega-3 fatty acids (EPA and DHA) for adults which is 0.25 g per day).

However, an alternative to fish oils is wished to obtain the recommended intake of EPA and DHA, i.e. since vegetarians and vegans do not eat fish oil and since fish oil has a taste and smell disliked by many. There is an increasing demand for vegetarians and vegans in need of supply of the beneficial polyunsaturated fatty acids EPA and DHA as an alternative to fish oil. Plant oil is not a suitable alternative as ALA present in plant oil is poorly converted to EPA and DHA. The conversion is so low that unsatisfactory high amounts of plant oil would be necessary to digest in order to provide a sufficient amount of converted EPA and DHA. Thus, plant oils in general are not a suitable solution as an alternative to fish oil in supplying the EPA and DHA.
The SDA component may be in the form of either an oil or a concentrate. In a preferred embodiment, the SDA component is in the form of an oil. In an embodiment of the invention, the SDA component is from a vegetable source.
For example, the SDA component may be Echium oil. Echium oil comprises a minimum of 12% SDA and has a SDA content between 12-15% by weight.
In another embodiment, the SDA component may be a concentrate comprising even higher amount of SDA, such as from 30-70% SDA, preferably about 40-60% SDA.
The SDA component may also be gene modified oil, for example a gene modified soyabean oil, comprising amounts of SDA in the range from 20-40 % SDA. Gene modified soybean oil is a biotechnological product that changes the oil composition in the soybean, in order to produce oils rich in SDA.
In a preferred embodiment, the SDA component is a natural oil. For the past years, consumers perceptibility has been increasing towards that ingredients consumed or digested should be natural, i.e. unmodified. Thus, there is an increasing demand to prepare products comprising all natural ingredients. Echium oil is a natural oil with a high content of SDA and is therefore very suitable as the SDA component according to the present invention. However, other natural oils may be used in the lipid composition according to the invention.
In an embodiment of the invention, the edible lipid composition comprises the SDA component in an amount of at least 25% by weight. The lipid composition may for example comprise the SDA component in an amount of at least 30% by weight, preferably at least 35% by weight, such as at least 40% by weight, even more preferably at least 45% by weight, such as at least 50% by weight, for example at least 55% by weight. In a preferred embodiment, the lipid composition comprises more than 50% of the SDA component, i.e. the SDA component is the predominant lipid source in the lipid composition.

The edible lipid composition according to the present invention is preferably comprising the SDA component in an amount of from 25 to 80% by weight, for example from 30 to 75% by weight, preferably from 40 to 72% by weight, such 45 to 70% by weight, such as from 50 to 70% by weight, even more preferably from 55 to 65% by weight.

The present inventors have found out that when SDA is eaten as a food supplement in combination with olive oil, the SDA is converted to EPA and DHA in an amount of above 20% conversion. For EPA and DHA to have an effect in preventing for example cardiovascular diseases, a certain amount of EPA and DHA is necessary to be converted/obtained. Thus, a certain amount of SDA has to be ingested by a human being such that enough EPA and DHA can be produced to have an effect.

In an embodiment of the invention, the edible lipid composition comprises SDA in an amount of at least 3 g SDA per 100 ml lipid composition, such as at least 4 g SDA per 100 ml lipid composition, preferably at least 5g/100 ml, such as at least 6 g/100 ml. Around 3 g/day of SDA should be consumed to produce the same effect as 1 g/day of EPA, based on the calculated relative efficiency of red blood cell incorporation of EPA. In a further embodiment, the lipid composition comprises at least 3% by weight SDA, such as at least 4% by weight of SDA, preferably at least 6% by weight SDA.

As discussed above, the SDA component may be derived from various sources, including various vegetable sources. SDA is for example present in hemp seed oil. However, hemp seed oil comprises components which are wished to be avoided, for example hemp seed oil comprises cannabinoids. In Struempler, R. E. et al "A positive Cannabinoids Workplace Drug test Following the Ingestion of Commercially Available Hemp Seed Oil", from Journal of Analytical Toxicology, Vol. 21, July/August 1997 is described a study where a test person ingests a commercially available cold-pressed hemp seed oil twice a day for 4½ days. From urine tests, it was shown that the amount of 11-nor-Δ9-tetrahydrocannabinol carboxylic acid (9-THCA) increased in the urine from every amount of hemp seed oil ingested. 9-THCA is the major pharmacologically active component of the marijuana plant (*Cannabis sativa)*. Thus, by ingestion of hemp seed oil, a person would be tested positive in a drug test which is wished avoided by the present invention.

Thus, in a preferred embodiment according to the present invention, the SDA component is not a hemp seed oil.

Besides, hemp seed oil comprises a low amount of SDA, such as 1-2% which will result in a low conversion of the important fatty acid EPA. Furthermore, the linoleic acid (omega-6 fatty acid) content in hemp seed oil is high (50-70%) which is not preferred. In the lipid composition according to the present invention, the omega-3 fatty acid content should be high, not the omega-6 fatty acid content. The omega-6 fatty acids, such as linoleic acid (LA - C18:2), gamma-linolenic acid (GLA - C20:3), and arachidonic acid (AA - C20:4) are not part of the pathway of conversion to eicosapentaenoic acid (EPA) and docosahexaenoic acid (DHA). On the contrary, omega-3 fatty acids may be converted to EPA and DHA, but different omega-3 fatty acids have different conversion rate. Where alpha-linolenic acid (ALA - C18:3) has been found to poorly convert to EPA, stearidonic acid (SDA - C18:4) has been found to have a high conversion rate to EPA.

Thus, in an embodiment of the invention, the SDA component comprises linoleic acid (LA) in amounts below 10% by weight, such as below 8% by weight, preferably below 7% by weight.

However, the lipid composition according to the present invention should not be free of omega-6 fatty acids, since some of the omega-6 fatty acids have other beneficial properties, e.g. gamma-linolenic acid (GLA) which is an essential fatty acid for the skin and has anti-inflammatory properties. GLA in combination with SDA also has the beneficial effect that blood cell EPA levels raise more effectively than EPA alone.

In an embodiment according to the invention, the SDA component comprises omega-3 fatty acids in an amount of at least 4% by weight, such as from 4 to 40% by weight, for example from 5 to 35% by weight, such as from 10 to 30% by weight, preferably from 15 to 28% by weight.

In another embodiment of the present invention, the SDA component comprises GLA in an amount of at least 5% by weight, preferably at least 8% by weight, such as 8-12% by weight.

GLA may for example be present in the SDA component in an amount from 5 to 20%, such as from 10 to 15%.

### Olive oil:

The present invention comprises an olive oil.

The olive oil present in the lipid composition according to the invention is preferably present in an amount of at least 25% by weight. For example olive oil is present in an amount of at least 30% by weight, even more preferably at least 35% by weight, for example at least 40% olive oil by weight of the lipid composition.

In a further embodiment, the lipid composition comprises olive oil in an amount of from 25 to 60% by weight of the lipid composition.

For example, the lipid composition comprises olive oil in an amount of from 25 to 60% by weight of the lipid composition, such as from 25 to 50% by weight, preferably from 30-45% by weight, such as from 35-45% by weight.

As discussed above, stearidonic acid (SDA) is converted to EPA and DHA, which are polyunsaturated fatty acids which are beneficial for a human being and can prevent the development of cardiovascular diseases and other life-style diseases. When SDA is administrated together with olive oil, a synergistic effect is obtained, such that olive oil protects SDA from oxidation in the oil and protects EPA and DHA converted from the SDA from oxidation in the human body which can cause oxidative stress.
Thus, the more SDA present in the lipid composition, the more important the olive oil becomes, as it protects long chain fatty acids from oxidizing.

The protective effect of olive oil to prevent long chain fatty acids from oxidising is primarily due to the large content of polyphenols present in the olive oil, since polyphenols have an antioxidative effect. Different olive oils may have a different content of polyphenols and it is important for the present invention that the olive oil comprises a high amount of polyphenol such as at least 250 mg polyphenols per kg olive oil. Polyphenols from olive oil contribute to the protection of blood lipids from oxidative stress. However, this effect is only for olive oils comprising 250 mg polyphenols per kg olive oil or more.

Besides having a protective antioxidative effect, olive oil also increses the absorption of the SDA in the body and thus the conversion of SDA to EPA and DHA.

The edible lipid composition according to the present invention must comprise both the SDA component and the olive oil with polyphenols in certain amounts. A certain amount of SDA is important in order to be able to convert enough EPA and DHA for the lipid composition to be administrated to a human being in a preferred dosis. The preferred amount of omega-3, EPA, and DHA in the blood is above 8%. Further, olive oil with a certain amount of polyphenols should be comprised in the edible lipid composition according to the present invention, in an amount such that the polyphenols provide an antioxidative effect and increase the absorption of SDA and thus the convertion of SDA to EPA and DHA.

In an embodiment of the invention, the edible lipid composition comprises olive oil and SDA component, wherein the ratio between the olive oil and SDA component is from 2:8 to 3:2, preferably 3:8 to 1:1, such as from 1:2 to 1:1, preferably 4:6.

In a further embodiment of the invention, the edible lipid composition comprises olive oil and SDA component, wherein the ratio between the olive oil and the SDA component is from 3:8 to 3:2, such as from 4:8 to 4:3; preferably 5:8 to 4:4, even more preferably from 4:6 to 6:4, such as from 45:55 to 50:50.

In a further embodiment, the olive oil polyphenols in the edible lipid composition is 100 to 350 mg polyphenols per kg SDA component (e.g. SDA oil), such as 125-300 mg polyphenol per kg SDA component, preferably 140 to 250 mg polyphenol per kg SDA component. In a most preferred embodiment, edible lipid composition comprises 150 to 200 mg polyphenols per kg SDA component. The optimum amount of polyphenols is 160 mg per kg SDA component.

Polyphenols in olive oil have an EFSA claim of protecting LDL particles from oxidative damage when 5 mg hydroxytyrosol and derivates are consumed daily. Also, polyphenols are believed to have a role in preventing cardiovascular diseases, or at least reduce the risk of obtaining cardiovascular diseases, through an improvement in vascular function and a modulation of inflammation. Polyphenols are powerful anti-inflammation agents blocking inflammatory and tissue-damaging enzymes. Polyphenols, such as those from olives (tyrosol, hydroxytyrosol etc.), also possess antioxidant properties protecting the cells and the blood lipids from oxidative stress proportionally to intake.

A high amount of polyphenols in an olive oil is also believed to increase the amount of the high density lipoprotein fraction (HDL) which is the beneficial lipoprotein, as compared to olive oils with a low amount of polyphenols which provides a higher amount of the unwanted low density lipoprotein (LDL).

In an embodiment according to the present invention, the olive oil is a virgin olive oil, preferably an extra virgin olive oil. Extra virgin olive oil comprises a higher amount of polyphenols as compared to a virgin olive oil and a general olive oil.

In an embodiment of the invention, the lipid composition comprises olive oil comprising polyphenols in an amount of at least 250 mg/kg, and preferably at least 300 mg/kg, such as at least 350 mg/kg.

In a preferred embodiment, the olive oil comprises polyphenols in an amount of from 250 to 700 mg/kg, such as from 300 to 600 mg/kg, preferably from 300 to 550 mg/kg, such as from 350-550 mg/kg.

The amount of polyphenols in the olive oil is preferably in the range of 0.025 to 1% by weight and will preferably be in the range of 0.03 to 0.5% by weight such as in the range of 0.035 to 0.1% by weight.

Thus, the olive oil present in the edible lipid composition according to the present invention comprises polyphenol in the range of 0.030 to to 0.01% by weight.

Further, a combination of omega-3 long chained fatty acids and olive oil are belived to help to subdue hyperactive monocyte cells in the immune system while simultaneously counteracting oxidation of LDL-cholesterol. These are two major risk factors in the process of atheroscelrosis. Thus, the composition according to the present invention comprising a combination of SDA and olive oil is believed to have an effect on atherosclerosis.

Besides from olive oil protects long chained fatty acids from oxidation and thus has an improved conversion of SDA to EPA and/or DHA, olive oil contains other beneficial ingredients, such as high amounts of oleic acid, polyphenols, and squalene.

For example, olive oil comprises oleic acid which is a monounsaturated omega-9 fatty acid (C18:1 n-9). By having a high amount of oleic acid present in the lipid composition, saturated fatty acids are beneficial replaced by the more beneficial oleic acid, which are favourable in the maintaince of normal LDL-cholesterol concentrations.

In an embodiment of the invention, the olive oil comprises at least 50% by weight oleic acid, such as at least by weight 55% oleic acid.

In a further embodiment of the invention, the lipid composition comprises oleic acid in an amount of at least 10% by weight, preferably at least 20% by weigh, such as at least 30% by weight.

Preferably, the lipid composition comprises oleic acid in an amount of 10-70% by weight, such as 15-60% by weight oleic acid, preferably 20-55% by weight oleic acid, for example 25-50% by weight oleic acid, such as 30-50% oleic acid by weight, even more preferably 35-45% oleic acid by weight

As mentioned earlier, polyphenols in olive oil have a protective effect against oxidation of EPA and DHA.

Olive oil contains several other health beneficial phenolic compounds, such as hydroxytyrosol and its secoiriodic derivates, and squalene. In addition, olive oil comprises tocopherols, sterols, and other natural antioxidants.

In an embodiment of the invention, the edible lipid comprises olive oil comprising squalene in an amount of at least 200 mg/kg.

Squalene is preferably present as phytosqualene and present in the olive oil in an amount of from 200 mg/kg to 15 g/kg, such as from 300 mg/kg to 12 g/kg, preferably in an amount of 400 mg/kg to 7 g/kg, such as 420 mg/kg to 1.2 g/kg, even more preferably from 450 mg/kg to 900 mg/kg, such as from 500 mg/kg to 800 mg/kg. In a preferred embodiment of the invention, the olive oil comprises 400-700 mg/kg squalene, preferably 400-450 mg/kg or 600-700 mg/kg squalene and in another embodiment the olive oil comprises from 800 mg/kg to 12 g/kg. Squalene is the major hydrocarbon in olive oil and squalene is believed to improve health. Further, squalene provides an improved protection of the skin when digested, by protecting the skin surface from lipid peroxidation due to external sources. Squalenes protective effect is due to scavenging singlet oxygen generated by ultraviolet light.

In another embodiment of the invention, the olive oil comprises from 0.2 to 1.0% by weight squalene, such as from 0.3 to 0.9% by weight squalene, preferably from 0.4 to 0.8% by weight squalene, even more preferably from 0.5 to 0,75% by weight squalene, such as from 0.5 to 0.7% by weight squalene.

The polyphenols in olive oil, responsible for the stability and flavour, is endorsed with pharmacological properties. Phenolic compounds such as hydroxytyrosol and oleuropein in extra virgin olive oil are powerful antioxidants, both in the oil and in the body. They have bioactive properties that support the effects of the Mediterranean diet being effective against oxidative stress associated health issues, including ageing, in a dose-dependen manner. Oxidative stress is defined as an imbalance between the oxidant and the antioxidant systems of the body in favour of the oxidants.

Olives and olive-derived products have beneficial health effects, particularly on the cardiovascular system. These benefits are due to a combination of a high content of oleic acid and a variety of polyphenol compounds found in olive-derived products. The phenolic compounds present in olive oil are strong antioxidants and radical scavengers. Virgin olive oil is well known for its high content of phenolic substances such as oleuropein, hydroxytyrosol (2-(3,4-dihydroxyphenyl)ethanol), and tyrosol (2-(4-hydroxyphenyl)ethanol). Hydroxytyrosol and its derivates have shown in in vivo studies to have various biochemical roles including anti-inflammatory by inhibiting aggregation of platelets, prevent accumulation of the pro-aggregant thromboxane in human serum, inhibiting LOX activity and the production of pro-inflammatory molecules, such as leukotrines, i.e. leukotriene B, and as antioxidants by prevent low density lipoprotein oxidation. The in vivo oxidation of LDL is linked to the formation of atherosclerotic plaques, which are postulated to contribute to the development of coronary heart disease.

### A further oil:

The edible lipid composition according to the present invention may in an embodiment comprise one or more further oil. In some instances, the conversion of EPA and DHA from SDA may not be sufficient and then, a further oil comprising EPA and/or DHA may be included in the lipid composition in order for the amount of converted EPA and DHA from the lipid composition to meet recommended amounts of EPA and DHA made by authorities to have a healthy effect, i.e. to reduce the risk of develop cardiovascular diseases.
For general health and nutrition, most authorities are recommending 200 mg to 650 mg of EPA and DHA per day. European Food Safety Authority (EFSA) has recently approved the following quantitative claims for EPA and DHA (Omega-3 from fish):
- 2 g daily intake of DHA and EPA contributes to the maintenance of normal blood triglyceride concentrations. However, maximum 5 g EPA and DHA per day is recommended.
- 3 g daily intake of DHA and EPA contributes to the maintenance of normal blood pressures. However, maximum 5 g EPA and DHA per day is recommended.
According to EFSA, it is safe to consume up to 5 gram Omega-3 daily.

In an embodiment of the invention, the lipid composition comprises from 25 to 40% by weight of the SDA component, 30 to 50% by weight of olive oil and 20 to 35% by weight of one or more further oil.

The further oil is preferably an oil comprising high amounts of EPA and DHA, such as 5% by weight or more EPA and DHA, preferably 7% by weight or more EPA and DHA, such as 10% by weight or more EPA and DHA, even more preferably 12% by weight or more EPA and DHA, such as 15% by weight or more of EPA and DHA. The further oil is preferably derived from a plant source such as an alga oil or is ethylesters of EPA and/or DHA. The further oil may also preferably be in the form of ethylesters of EPA and DHA.

### Alga oil:

The further oil may be alga oil and in an embodiment of the invention, the further oil is alga oil. Alga oil is a vegetarian source rich in DHA or EPA. Alga oil is beneficial to use in combination with an olive oil and SDA component in the lipid composition according to the present invention when there is a higher need for EPA than what can be converted from SDA. Alga oil is an ideal alternative to those who wish to avoid fish or soy products. By adding alga oil to the lipid composition comprising olive oil and a SDA component, the lipid composition becomes a full nutrition as alternative to fish oil, as alga oil would add EPA or DHA such that the recommended values of EPA and DHA are met by the lipid composition. A pure alga oil is however not an alternative to fish oil, since alga oil contains either EPA or DHA and not the combination. Thus, alga oil is only suitable as an additive and not a complete nurtrition.

Ideally, vegetarians and vegans who are seeking to supplement with omega-3 for optimal heart health should seek oils containing EPA and DHA.

Alga oil may for example be present in the edible lipid composition according to the present invention in an amount of from 0 to 40% by weight, such as from 5 to 40% by weight, preferably from 10 to 35% by weight, such as from 15-30% by weight, even more preferably from 20 to 30% by weight.

In an embodiment of the invention, the lipid composition comprises from 25 to 40% by weight of the SDA component, 30 to 50% by weight of olive oil and 20 to 35% by weight of alga oil.

In a further embodiment of the invention, the lipid composition comprises 30% by weight of the SDA component, 40% by weight of olive oil and 30% by weight of alga oil.

### Ethyl esters of EPA and/or DHA:

The further oil may be ethyl esters of EPA and/or DHA.
Ethyl esters of EPA and DHA are already digested fatty acids which are ready for absorption. Thus, ethyl esters of EPA and/or DHA are an industrial source of omega-3 fatty acids.

EPA and DHA ethyl esters are a chemically modified form of fatty acids for the purpose of producing concentrates with high EPA and DHA content. During this process the glycerol backbone is removed from EPA and DHA. The free fatty acids are then esterified to form ethyl esters. Another method is to first use ethylation to concentrate EPA and DHA before the ethyl esters are broken down and reconverted to triglyceride. Both these forms are classified as esters. In the ethyl ester, the fatty acids are esterified into a ethanol backbone.
Some people have a poor absorption of triglycerides from the diet and some people do not have any absorption of triglycerides from the diet. The inventors of the present invention believe that this is due to a decrease or lack of lipases in the small intestine or other problems in the small intestine. In order to overcome this problem of poor digestion of triglyceride, ethyl esters of EPA and/or DHA can be added to the nutritional composition of the present invention.

In a preferred embodiment, the ethyl esters of EPA and/or DHA is from a vegetarian source.

Ethyl esters of EPA and/or DHA may for example be present in the edible lipid composition according to the present invention in an amount of from 0 to 40% by weight, such as from 5 to 40% by weight, preferably from 10 to 35% by weight, such as from 15-30% by weight, even more preferably from 20 to 30% by weight.

In an embodiment of the invention, the lipid composition comprises from 25 to 40% by weight of the SDA component, 30 to 50% by weight of olive oil and 20 to 35% by weight of ethyl esters of EPA and/or DHA.

In a preferred embodiment, the lipid composition comprises both alga oil and ethyl esters of EPA/DHA, such as 5 to 40% by weight of alga oil and ethyl esters of EPA and/or DHA, for example 2.5 to 20% by weight of alga oil and 2.5 to 20% by weight of ethyl esters of EPA and/or DHA.

### Method:

The method of preparing the lipid composition according to the present invention comprises mixing of an olive oil comprising polyphenols in an amount of at least 250 mg/kg and a SDA component comprising at least 6% SDA and optionally a further oil, wherein the ratio between the olive oil and the SDA component is from 3:8 to 3:2. The blending of the oil components are performed to achieve optimal omega-3 and protection against oxidation. The mixing of olive oil and the SDA component is made by standardized oil blending procedures.

### Use:

The edible lipid composition according to the present invention comprises the omega-3 fatty acid stearidonic acid (SDA) in high amounts in combination with olive oil, wherein the olive oil increases the absorption of the SDA in the body and thus conversion of SDA to EPA and DHA.

EPA has anti-inflammatory effects. Studies have suggested that EPA has superior lipid management properties, lowering cholesterol and contributing to heart and cardiovascular health. EPA is also thought to have strong neuro-protective properties, positively affecting mental conditions such as schizophrenia, depression, and overall mood. Recent studies suggest that when used in combination, EPA boosts the effectiveness of proven blockbuster drugs.

Also, EPA and DHA will lower triglyceride levels and high triglyceride content can lead to type II diabetes and raise the risk of heart disease, while only EPA increases good HDL cholesterol and lowers bad LDL cholesterol.

Further, it is known in the art that intake of long chained omega-3 polyunsaturated fatty acids may reduce the risk of developing cancer (not alpha-linolenic acid - ALA), such as breast cancer, by up to 14%

Thus, EPA and DHA are known to reduce or prevent the risk of developing atherosclerosis, thrombosis, and other cardiovascular diseases as well as other inflammatory diseases such as psoriasis, diabetes, arthritis, cancer, osteoporosis, and astma. Omega-3 fatty acids reduce the growth rate of atherosclerosis plaque and therefore they have inflammatory reducing quality and characteristics, since injuries in the atherogenic process are mediated by pro-inflammatory reactions.

Thus in an aspect of the invention, the edible lipid composition is used in administration to an animal or human for preventing or reducing the risk of developing cardiovascular diseases, coronary thrombosis, atherosclerosis, cancer, diabetes (especially diabetes II), alzheimers, arthritis, rheumatism, osteoporosis, psoriasis, or astma.

The lipid composition according to the present invention may be used as a supplement to the regular diet or as a component in a diet, e.g. as a component in an oil-in-water or water-in-oil emulsion in food products.

The edible lipid composition may be incorporated into various food products, which incorporation may be achieved by for example emulsification by homogenization or by using an encapsulation technique, such as microencapsulation. The microencapsulation may be obtained by for example spray drying and freezing. By microencapsulation, oil drops are encapsulated inside a protein-carbohydrate matrix that protects the oil and creates a dry dispersible powder. By the emulsification method, small oil droplets are dispersed in the food product. The lipid composition may also be encapsulated in gelatine capsules which will enhance the organoleptic properties of the oil when swallowing. However, gelatine capsules are not suitable to be ingested by vegetarians or vegans.

In a preferred embodiment, the edible lipid composition according to the present invention is used in enriching food product with omega-3 fatty acids. The lipid composition may be incorporated into a range of foods, including milk based products, drinkable yoghurts, chocolate, breakfast cereals, and food bars.
In an embodiment of the invention, the invention relates to the use of the edible lipid composition according to the present invention as an ingredient in food products.
For example, the lipid composition according to the present invention may be used for enriching chocolate with omega-3 fatty acids. Thus, the present invention also relates to use of the edible lipid composition as an ingredient in chocolate. The lipid composition may also be used in food bars, where the lipid composition is incorporated as an omega-3 supplement to the regular diet. Lean fish products can be enriched with the lipid composition to achieve properties as fat fish products.
It should be noted that embodiments and features described in the context of one of the aspects of the present invention also apply to the other aspects of the invention.

The invention will now be described in further details in the following non-limiting examples.

### Examples

### Comparative Example 1 - Composition comprising SDA oil and olive oil

In the below table, different lipid compositions are shown. The lipid compositions all comprise an SDA oil and an olive oil.

**Table 1. Different lipid compositions comprising SDA oil and olive oil**

| Recipe | SDA oil (%-wt) | Olive oil (%-wt) | Alga oil (%-wt) | Omega-3 ethyl esters (%-wt) |
|---|---|---|---|---|
| A | 90 | 10 | | 0 |
| B | 70 | 25 | 5 | |
| C | 70 | 25 | | 5 |
| D | 70 | 25 | 2.5 | 2.5 |

The SDA oil used comprises the fatty acid profile mentioned below in table 2.

**Table 2. Fatty acid profile of SDA oil**

| Fatty acid | | % by weight (%-wt) |
|---|---|---|
| Oleic acid | | 12-15 |
| Linoleic acid (LA) | | 12-15.5 |
| Alpha-linolenic acid (ALA) | | 25-32.5 |
| Gamma- linolenic acid (GLA) | | 8-12 |
| Stearidonic acid (SDA) | | 10-12 |
| Other fatty acids | | 12-14 |

The olive oil used is an extra virgin olive oil comprising the fatty acid profile mentioned in table 3:

**Table 3. Fatty acid profile of olive oil**

| Fatty acids | Amount (%) |
|---|---|
| Myristic acid | 0.01-0.02 |
| Palmitic acid | 10-15 |
| Palmitoleic acid | 0.9-2 |
| Stearic acid | 3-3.2 |
| Oleic acid | 60-66 |
| Linoleic acid | 5-10 |
| Linolenic acid | 0.6-0.7 |
| Other fatty acids | 5 |

The olive oil used comprises 400 mg polyphenols per kg olive oil.

The compositions A, B,C, and D are prepared by mixing the SDA oil with the olive oil and further oil by standardized oil blending procedures.

### Example 2 - Composition comprising SDA concentrate and oil.

In the below table, different lipid compositions according to the present invention comprising an SDA concentrate and olive oil are shown.

**Table 4. Different lipid compositions comprising SDA concentrate and olive oil**

| Recipe | SDA concentrate (%-wt) | Olive oil (%-wt) | Alga oil (%-wt) | Omega-3 ethyl esters (%-wt) |
|---|---|---|---|---|
| E | 70 | 30 | 0 | 0 |
| F | 55 | 40 | 5 | |
| G | 55 | 40 | 0 | 5 |

The olive oil used is the same as in example 1, and the SDA concentrate comprises about 60% SDA (60 g SDA per 100 gram).

The compositions E to G are prepared by mixing the SDA concentrate with the olive oil by standardized oil blending procedures.

### Example 3 - Amount of SDA, EPA; DHA and polyphenols in lipid compositions

In the lipid composition described in A, B, F and G in example 1 and 2 above, the SDA and polyphenol content is measured and given in table 5 below. Further, the EPA, DHA, and DPA converted from the SDA is shown.

**Table 5. Amounts of various components**

| Recipe | SDA (g/100 ml) | EPA (g/100 ml) | DHA (g/100 ml) | DPA (g/100 ml) | Omega-3 (g/100 ml) | Polyphenols (mg/100 ml) |
|---|---|---|---|---|---|---|
| A | 9.1 | 3.4 | 1.7 | 0.6 | 5.6 | 40.0 |
| B | 7.3 | 4.4 | 2.2 | 0.7 | 7.4 | 100.0 |
| F | 40.0 | 3.4 | 1.9 | 0.6 | 6.4 | 160.0 |
| G | 20.5 | 4.8 | 2.4 | 0.8 | 7.9 | 160.0 |

### Example 4 - Comparison of different ratios between SDA oil and oil

Different lipid compositions were prepared as described in example 1 or 2 above where an SDA oil comprising 25% SDA and an olive oil comprising 400 mg/kg polyphenol were used, but where the ratio between SDA oil and olive oil varies. Below in table 6 is shown the amount of SDA oil and olive oil in different lipid compositions. Further, the EPA and DHA which is obtained from the lipid composition after conversion of SDA is given. Also, the polyphenol content in the lipid compostion is given.

Examples I-IV are not according to the invention.

**Table 6. Amounts of various components**

| SDA | SDA (%) | Olive oil (%) | Total omega -3 (%) | EPA (%) | DPA (%) | DHA (%) | Polyphenols (mg(kg) |
|---|---|---|---|---|---|---|---|
| I | 5 | 95 | 0.44 | 0.26 | 0.04 | 0.013 | 380 |
| II | 10 | 90 | 0.88 | 0.53 | 0.09 | 0.26 | 360 |
| III | 20 | 80 | 1.75 | 1.05 | 0.18 | 0.53 | 320 |
| IV | 30 | 70 | 2.63 | 1.58 | 0.26 | 0.79 | 280 |
| V | 40 | 60 | 3.50 | 2.10 | 0.35 | 1.05 | 240 |
| VI | 45 | 55 | 3.38 | 2.03 | 0.34 | 1.01 | 220 |
| VII | 50 | 50 | 3.75 | 2.25 | 0.38 | 1.13 | 200 |
| VII | 60 | 40 | 4.50 | 2.70 | 0.45 | 1.35 | 160 |
| VIII | 70 | 30 | 4.38 | 2.63 | 0.44 | 1.31 | 120 |
| IX | 80 | 20 | 5.00 | 3.00 | 0.50 | 1.50 | 80 |

The conversion rate of SDA to EPA and DHA is increased the more polyphenol is present in the lipid composition.

### Example 5 - Comparison of different amounts of SDA present in the SDA oil

Different lipid compositions were prepared as described in example 1 or 2 above having 50% of an SDA oil and 50% of an olive oil, the olive oil comprising 400 mg/kg polyphenol and where lipid compositions varies in the amount of SDA present in the SDA oil. Below in table 7 is shown the amount of SDA oil, the amount of SDA present, and the amount of olive oil in different lipid compositions. Further, the EPA and DHA which is obtained by the lipid composition after conversion of SDA is given. Also, the polyphenol content in the lipid compostion is given.

**Table 7. Amounts of various components**

| SDA | SDA presen t in SDA oil (%) | SDA oil (%) | Olive oil (%) | Total omega -3 (%) | EPA (%) | DPA (%) | DHA (%) | Polyphen ols (mg(kg) |
|---|---|---|---|---|---|---|---|---|
| I | 3 | 50 | 50 | 0.38 | 0.23 | 0.04 | 0.11 | 200 |
| II | 5 | 50 | 50 | 0.63 | 0.38 | 0.06 | 0.19 | 200 |
| III | 7 | 50 | 50 | 0.88 | 0.53 | 0.09 | 0.26 | 200 |
| IV | 10 | 50 | 50 | 1.25 | 0.75 | 0.13 | 0.38 | 200 |
| V | 12 | 50 | 50 | 1.5 | 0.90 | 0.15 | 0.45 | 200 |

### Example 6 - Comparison of different amounts of polyphenols present in the olive oil

Different lipid compositions were prepared comprising 50% SDA oil having a 25% SDA content and 50% olive oil. The compositions were prepared as described in example 1 or 2 above. Below in table 8 is shown the amount of SDA oil and olive oil in different lipid compositions. Further, the polyphenol content in the lipid compositions used is shown. Further, the EPA and DHA which is obtained by the lipid composition after conversion of SDA is given. Also, the polyphenol content in the lipid composition is given.

**Table 8. Amounts of various components**

| | polyph enol presen t in olive oil (mg/k g) | SDA oil (%) | Olive oil (%) | Total omega -3 (%) | EPA (%) | DPA (%) | DHA (%) | Polyphen ols (mg(kg) |
|---|---|---|---|---|---|---|---|---|
| I | 100 | 50 | 50 | 1.2 | 0.7 | 0.12 | 0.36 | 50 |
| II | 200 | 50 | 50 | 1.5 | 0.9 | 0.15 | 0.45 | 100 |
| III | 300 | 50 | 50 | 1.8 | 1.1 | 0.18 | 0.54 | 150 |
| IV | 400 | 50 | 50 | 2.1 | 1.3 | 0.21 | 0.63 | 200 |
| V | 500 | 50 | 50 | 2.4 | 1.4 | 0.24 | 0.72 | 250 |

The conversion rate of SDA to DHA and EPA is increased the more polyphenols are present in the olive oil.

### Example 7 - Comparison of different lipid compositions according to the invention

Different lipid compositions were prepared comprising 50% olive oil, different amounts of SDA oil (30% or 40%), and different amounts of alga oil, wherein the SDA oil comprises 25% SDA. The compositions were prepared as described in example 1 or 2 above. Below in table 9, the amount of SDA oil and olive oil in different lipid compositions is shown. Further, the polyphenol content in the lipid compositions is shown. Further, the EPA and DHA which is obtained by the lipid composition after conversion of SDA is given. The amount of polyphenol in the olive oil in 400 mg/kg.

The amount of SDA in the SDA oil is 25%.

Examples VII and IX are not according to the invention.

**Table 9. Amounts of various components**

| | SDA oil (%) | Olive oil (%) | Algae oil (%) | EPA + Omeg a concen trate | Total omega -3 (%) | EPA (%) | DPA (%) | DHA (%) | Polyphenols (mg(kg) |
|---|---|---|---|---|---|---|---|---|---|
| VI | 40 | 50 | 10 | 0 | 8.50 | 5.10 | 0.85 | 2.55 | 200 |
| VII | 30 | 50 | 20 | 0 | 13.88 | 8.33 | 1.39 | 4.16 | 200 |
| VIII | 40 | 50 | 0 | 10 | 11.50 | 6.90 | 1.15 | 3.45 | 200 |
| IX | 30 | 50 | 0 | 20 | 19.88 | 11.93 | 1.99 | 5.96 | 200 |

The Algae oil used comprises about 60% EPA + DHA.

### Example 8 - Oxidative stability test of olive oil with polyphenols

The effect of olive oil comprising polyphenols on oxidative stress of long chain fatty acids has been analysed. Two oil mixes have been made,
A) one oil mix comprising an oil with omega-3 fatty acids and vitamin E and
B) a second oil mix comprising the same oil with omega-3 fatty acids as in a) in combination with an olive oil having a content of polyphenols of 400 mg/kg.
The oxidation stability of both oil mixes A) and B) has been analysed by the oil stability index (OSI), which is a method determining the relative resistance of fats and oils to oxidation.
In figure 1 the oxidative stability of both oil mixes is shown.
From figure 1, it is shown that addition of an olive oil comprising polyphenols will increase the oxidative stability of an oil comprising omega-3 fatty acids, ie. the omega-3 long chain fatty acids, (EPA and DHA) in the oil is protected against oxidation. Figure 1 shows that the mixture of omega-3 fatty acid oil and olive oil are clearly more stable (35 hours) against lipid oxidation than an omega-3 fatty acid oil without olive oil, but stabilized with vitamin E (tocopherol) (16 hours). Thus, there is a synergistic protective effect of polyphenols on oxidative unstable long chain polyunsaturated fatty acids.

Since all fats and oils are prone to oxidation, a vegetable oil comprising Stearidonic acid (SDA) is also prone to oxidation. Thus, the antioxidative effect of polyphenols in olive oil will also occur on SDA oil and on EPA and DHA inside our body.

## Claims

1. An edible lipid composition comprising olive oil and a stearidonic acid component (SDA component), wherein said SDA component comprises stearidonic acid (SDA) in an amount of at least 6% by weight and wherein said olive oil comprises polyphenols in an amount of at least 250 mg per kg olive oil and wherein the ratio between the olive oil and the SDA component is from 3:8 to 3:2.

2. The edible lipid composition according to claim 1, wherein the lipid composition comprises one or more further oil.

3. The edible lipid composition according to claim 2, wherein the further oil is an alga oil or ethylesters of EPA and/or DHA.

4. The edible lipid composition according to any of the preceding claims, wherein olive oil is present in an amount of at least 25%.

5. The edible lipid composition according to any of the preceding claims, wherein the SDA component is present in an amount of at least 30% by weight.

6. The edible lipid composition according to any of the preceding claims, wherein the SDA component comprises SDA in an amount of at least 10% by weight.

7. The edible lipid composition according any of the preceding claims, wherein the lipid composition comprises SDA in an amount of at least 3 g SDA per 100 ml lipid composition.

8. The edible lipid composition according to any of the preceding claims, wherein the SDA component comprises GLA in an amount of at least 5% by weight .

9. The edible lipid composition according to any of the preceding claims, wherein the SDA component comprises linoleic acid (LA) in amount below 10% by weight.

10. The edible lipid composition according to any of the preceding claims, wherein the lipid composition comprises oleic acid is an amount of at least 10% by weight.

11. The edible lipid composition according to any of the preceding claims, wherein the olive oil comprises polyphenol in the range of 0.025 to 1% by weight.

12. An edible lipid composition according to any of the preceding claims, wherein the olive oil comprises squalene in an amount of at least 200 mg/kg.

13. An edible lipid composition according to any of the preceding claims, wherein the SDA component is not Hemp seed oil.

14. A method of preparing the lipid composition according to any of the claims 1 to 13, wherein the method comprising mixing of an olive oil comprising polyphenols in an amount of at least 250 mg per kg olive oil and a SDA component comprising at least 6% stearidonic acid and optionally a further oil.

15. The edible lipid composition according to any of claims 1 to 13 for use in administration to an animal or human for preventing or reducing the risk of developing cardiovascular diseases, coronary thrombosis, atheroschlerosis, cancer, diabetes, rheumatism, alzheimers, arthritis, rheumatism, osteoporosis, psoriasis or astma.

## Patentansprüche

1. Essbare Lipidzusammensetzung umfassend Olivenöl und eine Stearidonsäurekomponente (SDA-Komponente), wobei die SDA-Komponente Stearidonsäure (SDA) in einer Menge von zumindest 6 Gew.-% umfasst und wobei das Olivenöl Polyphenole in einer Menge von zumindest 250 mg pro kg Olivenöl umfasst und wobei das Verhältnis zwischen dem Olivenöl und der SDA-Komponente bei 3:8 bis 3:2 liegt.

2. Essbare Lipidzusammensetzung nach Anspruch 1, wobei die Lipidzusammensetzung ein oder mehrere weitere Öle umfasst.

3. Essbare Lipidzusammensetzung nach Anspruch 2, wobei das weitere Öl ein Algenöl oder Ethylester von EPA und/oder DHA ist.

4. Essbare Lipidzusammensetzung nach einem der vorstehenden Ansprüche, wobei Olivenöl in einer Menge von zumindest 25 % vorhanden ist.

5. Essbare Lipidzusammensetzung nach einem der vorstehenden Ansprüche, wobei die SDA-Komponente in einer Menge von zumindest 30 Gew.-% vorhanden ist.

6. Essbare Lipidzusammensetzung nach einem der vorstehenden Ansprüche, wobei die SDA-Komponente SDA in einer Menge von zumindest 10 Gew.-% umfasst.

7. Essbare Lipidzusammensetzung nach einem der vorstehenden Ansprüche, wobei die Lipidzusammensetzung SDA in einer Menge von zumindest 3 g SDA pro 100 ml Lipidzusammensetzung umfasst.

8. Essbare Lipidzusammensetzung nach einem der vorstehenden Ansprüche, wobei die SDA-Komponente GLA in einer Menge von zumindest 5 Gew.-% umfasst.

9. Essbare Lipidzusammensetzung nach einem der vorstehenden Ansprüche, wobei die SDA-Komponente Linolsäure (LA) in einer Menge unter 10 Gew.-% umfasst.

10. Essbare Lipidzusammensetzung nach einem der vorstehenden Ansprüche, wobei die Lipidzusammensetzung Oleinsäure in einer Menge von zumindest 10 Gew.-% umfasst.

11. Essbare Lipidzusammensetzung nach einem der vorstehenden Ansprüche, wobei das Olivenöl Polyphenol in dem Bereich von 0,025 bis 1 Gew.-% umfasst.

12. Essbare Lipidzusammensetzung nach einem der vorstehenden Ansprüche, wobei das Olivenöl Squalen in einer Menge von zumindest 200 mg/kg umfasst.

13. Essbare Lipidzusammensetzung nach einem der vorstehenden Ansprüche, wobei die SDA-Komponente nicht Hanfsamenöl ist.

14. Verfahren zum Herstellen der Lipidzusammensetzung nach einem der Ansprüche 1 bis 13, wobei das Verfahren ein Mischen eines Olivenöls umfassend Polyphenole in einer Menge von zumindest 250 mg pro kg Olivenöl und einer SDA-Komponente umfassend zumindest 6 % Stearidonsäure und gegebenenfalls eines weiteren Öls umfasst.

15. Essbare Lipidzusammensetzung nach einem der Ansprüche 1 bis 13 zur Verwendung bei der Verabreichung an ein Tier oder Menschen zum Vorbeugen oder Reduzieren des Risikos des Entwickelns von Herz-Kreislauf-Erkrankungen, Koronarthrombose, Atherosklerose, Krebs, Diabetes, Rheumatismus, Alzheimer-Krankheit, Arthritis, Rheumatismus, Osteoporose, Psoriasis oder Asthma.

## Revendications

1. Composition lipide comestible comprenant de l'huile d'olive et une composante d'acide stéaridonique (composante SDA), ladite composante SDA comprenant de l'acide stéaridonique (SDA) dans la mesure d'au moins 6% en poids, et ladite huile d'live comprenant des polyphénols dans la proportion d'au moins 250 mg par kg d'huile d'olive, et le ratio huile d'olive / composante SFA étant compris entre 3/8 et 3/2.

2. Composition lipide comestible selon la revendication 1, la composition lipide comprenant une ou plusieurs huiles supplémentaires.

3. Composition lipide comestible selon la revendication 2, les huiles supplémentaires étant de l'huile d'algue ou des esters éthyliques d'EPA et/ou de DHA.

4. Composition lipide comestible selon une quelconque des revendications précédentes, l'huile d'olive étant présente dans la proportion d'au moins 25%.

5. Composition lipide comestible selon une quelconque des revendications précédentes, la composante SDA étant présente dans la proportion d'au moins 30% en poids.

6. Composition lipide comestible selon une quelconque des revendications précédentes, la composante SDA comprenant du SDA dans la proportion d'au moins 10% en poids.

7. Composition lipide comestible selon une quelconque des revendications précédentes, la composition lipide comprenant du SDA dans une quantité d'au moins 3 g de SDA par 100 ml de composition lipide.

8. Composition lipide comestible selon une quelconque des revendications précédentes, la composante SDA comprenant du GLA dans une proportion d'au moins 5% en poids.

9. Composition lipide comestible selon une quelconque des revendications précédentes, la composante SDA comprenant de l'acide linoléique (LA) dans une quantité inférieure à 10% en poids.

10. Composition lipide comestible selon une quelconque des revendications précédentes, la composition lipide comprenant de l'acide oléique dans une quantité inférieure à 10% en poids.

11. Composition lipide comestible selon une quelconque des revendications précédentes, l'huile d'olive comprenant du polyphénol dans une proportion comprise dans la plage 0,025 à 1% en poids.

12. Composition lipide comestible selon une quelconque des revendications précédentes, l'huile d'olive comprenant du squalène dans une quantité d'au moins 200 mg/kg.

13. Composition lipide comestible selon une quelconque des revendications précédentes, la composante SDA n'étant pas de l'huile de chanvre.

14. Méthode de préparation de la composition lipide selon une quelconque des revendications 1 à 13, la méthode comprenant le mélange d'une huile d'olive comprenant des polyphénols dans une quantité d'au moins 250 mg/kg d'huile d'olive avec une composante SDA comprenant au moins 6% d'acide stéaridonique, et, en option, une autre huile.

15. Composition lipide comestible selon une quelconque des revendications 1 à 13 utilisée pour l'administration à un animal ou un être humain pour la prévention ou la réduction du risque de développement de maladies cardiovasculaires, de thromboses coronaires, d'athéroscléroses, du cancer, du diabète, de rhumatismes, de l'Alzheimer, de l'arthrite, de rhumatismes, de l'ostéoporose, du psoriasis, ou de l'asthme.
